# EUROPEAN PATENT SPECIFICATION

(11) **EP 2 491 386 B1**
(45) Date of publication and mention of the grant of the patent: **30.01.2019**
(21) Application number: 10770733.3
(22) Date of filing: 22.10.2010
(51) Int. Cl.: G01N 33/50, A61K 9/16, A61K 9/50

(54) **NESTED CELL ENCAPSULATION**
VERSCHACHTELTE ZELLEINKAPSELUNG
ENCAPSULATION DE CELLULES NICHÉES

(30) Priority: 22.10.2009 GB 0918564
(43) Date of publication of application: 29.08.2012
(73) Proprietor: Plasticell Ltd, London SW7 2BP (GB)
(72) Inventor: CHOO, Yen, London SW3 6PP (GB); JOHNSON, Christopher, James, Herne Hill London SE24 9NX (GB); ODENWÄLDER, Patrick, Klaus, 72074 Tubingen (DE); JAYASINGHE, Suwan, Nalin, London E11 4HZ (GB)
(74) Representative: Maschio, Antonio
(86) International application number: PCT/EP2010/006459
(87) International publication number: WO 2011/047870

(56) References cited:
- WO-A1-2007/023297
- US-A1- 2002 022 016
- US-A1- 2006 051 329
- Diaspro et al: "Single living cell encapsulation in nano-organized polyelectrolyte shells", Langmuir, vol. 18, no. 13 4 February 2002 (2002-02-04), pages 5047-5050, XP002621748, Retrieved from the Internet: URL:http://pubs.acs.org/doi/abs/10.1021/la 025646e [retrieved on 2011-02-10]
- HILLBERG ANNA L ET AL: "Biorecognition through layer-by-layer polyelectrolyte assembly: In-situ hybridization on living cells", BIOMACROMOLECULES, vol. 7, no. 10, October 2006 (2006-10), pages 2742-2750, XP002621749, ISSN: 1525-7797
- ODENWALDER PATRICK K ET AL: "Bio-electrosprays: A novel electrified jetting methodology for the handling and deployment of primary living organisms", BIOTECHNOLOGY JOURNAL, vol. 2, no. 5, May 2007 (2007-05), pages 622-630, XP002621750, ISSN: 1860-6768

## Description

The present invention relates to a method for encapsulating cells in a plurality of nested microcapsules. Labels are incorporated within each microcapsule, allowing identification of different cell populations according to the encapsulation or cell culture protocol. Moreover, the invention provides microencapsulated cells which comprise a plurality of microencapsulation layers and methods of tracking or identifying cells based on detection of microencapsulated labels.

Microencapsulation of cells has been proposed in the art since the 1960s. A review is provided in "Cell encapsulation: Promise and progress", Nature Medicine 9, 104 - 107 (2003). The scientific literature covers various encapsulation techniques and various encapsulated materials.

For example, the use of microencapsulated cells in medical applications was first proposed in 1964. Endocrine cells, islets, and hepatocytes were proposed to be encapsulated by microspheres formed by alginate/calcium complexes; see Chang, T. M. S., Artificial Cells, 1972, Springfield, Ill. Charles C. Thomas. In the 1980's, islets of Langerhans were encapsulated in alginate-poly-1-lysine-alginate capsules (Lim, F. and A. M. Sun, Microencapsulated islets as bioartificial endocrine pancreas. Science, 1980. 210: p. 908.) By using purer alginate and more viscous alginate solutions, researcher obtained microcapsules that were impermeable to normal serum immunoglobulin (Goosen, M. F. A., et al., Optimization of microencapsulation parameters: Semipermeable microcapsules as a bioartificial pancreas. Biotech. Bioeng., 1985.27: p. 146), thus insulating the cells from the body's immune response. See also, for example, US 4,353,888

Walsh et al, US 6,649,384 describe the use of a spinning disk atomiser to encapsulate cells, such as islet cells, for transplantation. This patent, as well as other published literature and patent documents, appears to cover mainly encapsulation for protection of cells from physical damage during handling or by attack from the immune system.

Techniques for culturing cells and methods for discovering and implementing techniques for regulation of cellular processes such as growth, differentiation, metabolic activity, and phenotypic expression are presented in our international application WO 2004/031369. According to the procedures described therein, "units" of cells, which comprise one or more cells cultured, for example, on a porous bead, are subjected to different growth conditions in a combinatorial split-pool procedure, which involves repeated splitting and re-pooling of cell cultures, to expose different cell units therein to different culture conditions.

When handling large numbers of cell units, their identity and/or cell culture history (for example, the chronology and the exact nature of a series of culture conditions that any one group or unit may have been exposed to) can become confused. WO2004/031369 describes improved methods for determining the identity and/or cell culture history of cell units.

In WO2007/063316 we describe methods for determining the activity of agents which act on a cell, using the split-pool procedure.

In WO2007/023297 we describe further improved methods for tagging cells in split-pool cell culture experiments, better to determine which reagents and nutrients a cell has been exposed to in achieving a particular state.

Encapsulation of living cells is known in the art, and has been pioneered for immuno-protection of transplanted cells. Generally, polymers useful for encapsulating cells for immuno-protection purposes, as known in the art, are useful in the present invention. For example, see Orive et al., (2203) Nature Medicine 9:104-107 and references cited therein.

Encapsulation of living material has been described using a jetting encapsulation technique. Many such techniques are known in the art, for example bio-electrospray jetting, aerodynamically-assisted bio-jetting and pressure-assisted cell jetting. Each of these techniques has been described as being useful for encapsulating living cells. For a general review of jetting technologies, see Jayasinghe, S., (2008) Regen. Med. 3:49-61, as well as US 6,649,384, US 2006/0051329 and US 4,353,888.

Encapsulation of cells has moreover been described using layer-by-layer (LbL) techniques. This technique involves the adsorption of multiple polyelectrolyte layers on to a surface to be coated and on to each other. Successive layers of cationic and anionic polyelectrolytes are used to form a multilayered structure. For example, see Peyratout and Daehne, (2004) Angew. Chem. Int. ed. 43:3762-3783. The application of LbL to encapsulating cells has been described, for example, by Leung et al., (2009) J Biomed Mater Res A 88:226-37. Leung *et al.* employ an alginate/poly-L-ornithine membrane to surround the cell, and coat this membrane with successive layers of polystyrene sulfonate and polyallylamine hydrochloride. The encapsulated cells are indicated to be useful for long-term graft transplantation.

Still further encapsulation techniques have been described which involve the use of microfluidic devices to encapsulate single cells or cell clusters. For example, see US2006/0051329.

Labelling cells which are exposed to split-pool culture techniques, or other techniques involving repeated rounds of culturing in different media, depends on being able to attach different labels to cells, depending on their exposure to various different media. This can be extremely laborious, especially in split-pool techniques where populations of cells are repeatedly pooled and re-split into different populations to sample a large number of different combinations of reagents. Moreover, it can be very difficult to follow the course of any one cell through the multiplicity of possible combinations of reaction conditions.

A number of patents and patent applications have been published covering multiple microcapsule "layers", which upon inspection turn out to be coatings rather than real layers. These coated encapsulations are created chemically, rather than by actual re-encapsulation to create a new layer. For example, see US 5,620,883. None of these methods has been suggested to be applicable to cell labelling.

Diaspro et al. (Langmuir, vol. 18, no. 13, 4 February 2002, pages 5047-5050) describe the encapsulation of cells using two different polyelectrolytes. One polyelectrolyte is labelled and the cells are stained with two different dyes.

Hillberg et al (Biomacromolecules, vol. 7, no. 10, October 2006, pages 2742-2750) provides encapsulation of cells in polyelectrolyte and the use of two different labels in the same layer.

### Summary of the Invention

The present invention provides a method for labelling cells or groups of cells by microencapsulating the cells such that the resulting microcapsule comprises two or more labels. Accordingly, there is provided a microcapsule comprising a living cell and a label.

The microcapsule may be any microcapsule which is can be used for encapsulating living cells, preferably without loss of function to the cell. For example, the microcapsule may be an alginate bead, for instance formed by microfluidic encapsulation or electro spraying, or a multilayered microcapsule comprising polyelectrolyte layers.

The label may be any label which is suitable for labelling living cells. Examples of labels are described below. The label is added during cell encapsulation. Advantageously, the label is incorporated into the microcapsule layer.

The cell may be a single cell, or a group of cells. Preferably, it is a cell unit.

In an example of the disclosure, there is provided a microcapsule comprising a living cell and a plurality of labels, wherein the cell is encapsulated within two or more capsule layers, and at least one label is associated with two or more of said microcapsule layers.

One or more labels is added as each microcapsule layer is added, thus labelling the microcapsules. A label may be incorporated into the microcapsule material itself, or enclosed within the microcapsule together with the cell. Each label is detectable. Preferably, every label can be detected at the same time, thus allowing multiple labels labelling a cell labelled to be recorded.

The invention provides a method for labelling a cell unit, comprising the steps of:
(a) providing one or more cell units each comprising one or more cells; and
(b) microencapsulating said cell unit(s) together with one or more first labels; and
(c) repeating the microencapsulation of step (b) together with a second label.

In step (c), the microcapsule obtained in step (b) is itself encapsulated. One or more second labels is added at this stage; the labels may be the same or different. The second label and the first label, moreover, are different.

Each microcapsule therefore contains, amongst other things, a label. In this manner, a cell is serially encapsulated a number of times, each time incorporating a different label, and thus retains a history of the encapsulation events to which it has been exposed.

The method of the invention is particularly advantageous when applied in cell culture protocols which involve exposure of cells to multiple culture conditions. If the encapsulation events are associated with exposure to identified reagents, the sequence of the reagents to which a cell has been exposed can be determined.

In a one aspect, therefore, there is provided a method for labelling a group of cells with a plurality of labels, comprising the steps of:
a) providing one or more cell units each comprising one or more cells;
(b) microencapsulating said cell units together with a first identifying label;
(c) optionally, mixing the cell units with one or more cell units which have been microencapsulated with a second identifying label;
(d) repeating step (b), using a third identifying label.

At least two of the first, second and third identifying labels are different, such that the different encapsulation events are associated with a specific label.

Cell units may microencapsulated individually, or in groups. In other words, a microcapsule may contain a single cell unit, or a plurality of cell units. For example, the microcapsule may contain 2, 3, 4, 5, 6 or more cell units.

Each of the cell units may itself be microencapsulated. Thus, each microcapsule may contain a plurality of microcapsules, each of which may contain a plurality of cell units. Alternatively, each microcapsule may contain a plurality of microcapsules, each of which comprises a single cell unit.

The distribution of numbers of cell units (or microcapsules) within microcapsules is advantageously homogenous, but it is expected that some variation may arise.

Advantageously, the method of the invention is used in the context of a split-pool procedure. In such a procedure, cells may be exposed to different culture conditions, pooled, split and optionally re-pooled, to sample the largest number of possible conditions.

In such an embodiment, the invention provides a method for labelling a group of cells which has been exposed to a plurality of culture conditions, comprising the steps of:
a) providing a first set of groups of cell units each comprising one or more cells, and exposing said groups to desired culture conditions;
(b) microencapsulating said cell units together with an identifying label;
(c) pooling two or more of said groups to form at least one pool;
(d) subdividing the pool to create a further set of groups of cell units;
(d) exposing said further groups to desired culture conditions;
(e) repeating steps (b) - (d).

Cell units may be single cells, or groups of cells, for example attached to microspheres or microporous microcarriers.

Microencapsulation can be performed by a jetting procedure. Preferred techniques include bio-electrospray jetting, aerodynamically-assisted bio-jetting and pressure-assisted cell jetting. Electrospray jetting is especially preferred. Alternative procedures include LbL adsorption of polyelectrolytes and microfluidic encapsulation.

The invention employs cell units. Such units may be single cells, but are advantageously colonies of two or more cells, which are arranged in such a form that they are resistant to disruption even during split pool procedures. For instance, the cells may be cultured on a solid substrate, such, as beads, as described in more detail below.

Typically the cell units used are microcarriers which are small enough to be encapsulated by a jetting method as described herein, such microcarriers also allow the use of cells for high-throughput screening (HTS) without any prior disruption of the cells units.

Labelling allows the following of the culture conditions to which the cells have been exposed; thus, any given cell unit can have its label read in order to determine how it has been derived from the starter cell pool or culture. Labelling may take any of a variety of forms, including nucleic acid labels, radiofrequency encoded tags, microsphere tags, bar-coded tags and micropshere tags. Microspheres are especially preferred.

The label may be selected from the group consisting of a virus, an oligonucleotide, a peptide, a fluorescent compound, a secondary amine, a halocarbon, a mixture of stable isotopes, a bar code, an optical tag, a bead, a quantum dot and a radiofrequency encoding tag. Two or more labels may even be selected from this group and used in combination to label a cell unit, for instance a bead comprising fluorescent compounds and/or quantum dots. Labelling and specific labels to be used with cell units are further discussed in our co-pending application WO2007/023297.

Cells may be cultured in cell units, each cell unit comprising one or more cells. In another embodiment, the cell units are single cells. The cell unit may comprise one or more cells adherent to or bound by a solid substrate. In a further embodiment, the solid substrate is a microcarrier or bead.

In one embodiment, the culture conditions include any physical or chemical medium in which cells are isolated and manipulated but suitably the reaction condition is a culture condition to which cells are exposed. Culture conditions include growth media, temperature regimes, substrates, atmospheric conditions, physical cell handling and the like. Growth media comprise natural and synthetic substances that nourish and affect the cells including but not limited to basal media, growth factors, nutrients, buffers, chemicals, drugs and the like. The reaction conditions may even comprise a screen of potential modulators of a cell signalling pathway.

An example of the disclosure relates to a microencapsulated cell unit comprising at least one cell unit encapsulated within a first microcapsule together with a first identifying label, said encapsulated cell unit being itself encapsulated within a second microcapsule together with a second identifying label.

As described above, each microcapsule may comprise a single cell unit, or a plurality of cell units. In one embodiment, therefore, the second microcapsule comprises a plurality of microencapsulated cell units, each of which may be individually microencapsulated or encapsulated with other cell units.

### Brief Description of the Figures

**Figures 1A** and **1B** are photomicrographs showing two different microcarriers encapsulated within a microcapsule.
**Figures 2A** and **2B** are photomicrographs showing similar microcarriers to those shown in

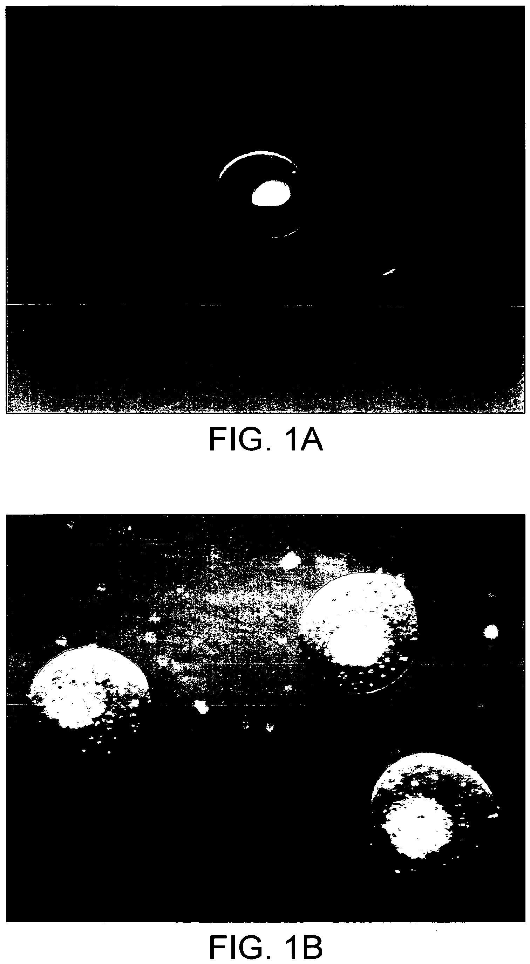

Figure 1, but encapsulated with two microcarriers to each microcapsule.

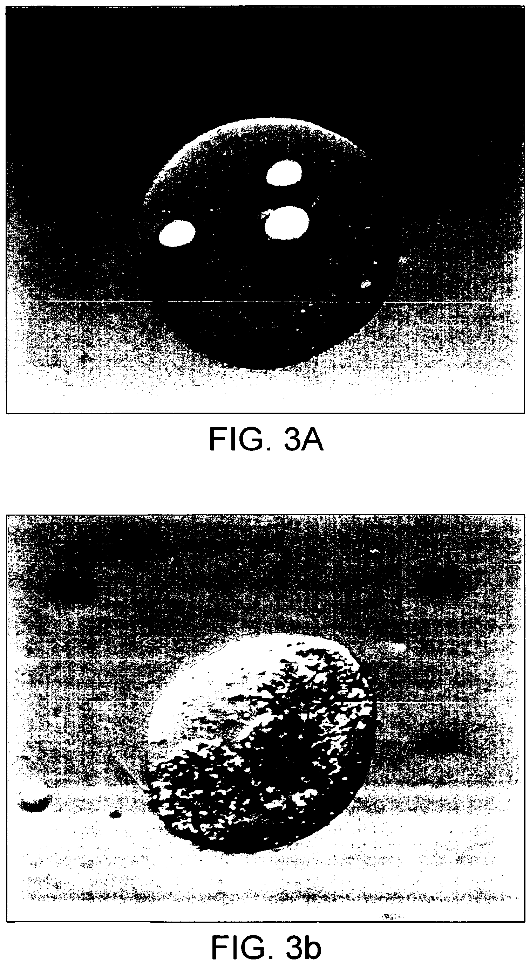

**Figures 3A** and **3B** are photomicrographs showing three microcarriers per microcapsule.

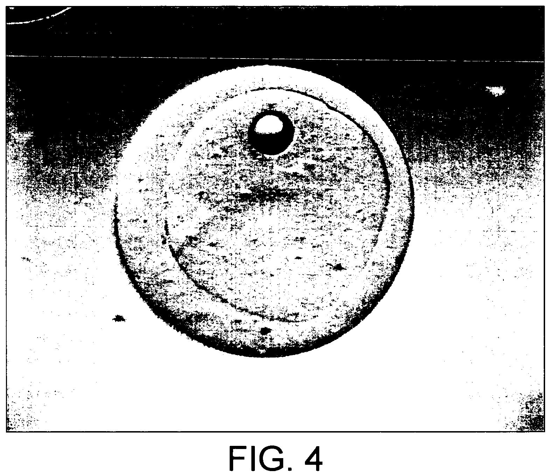

**Figure 4** is a photomicrograph showing a microcarrier encapsulated within a first microcapsule, itself encapsulated in a second microcapsule.

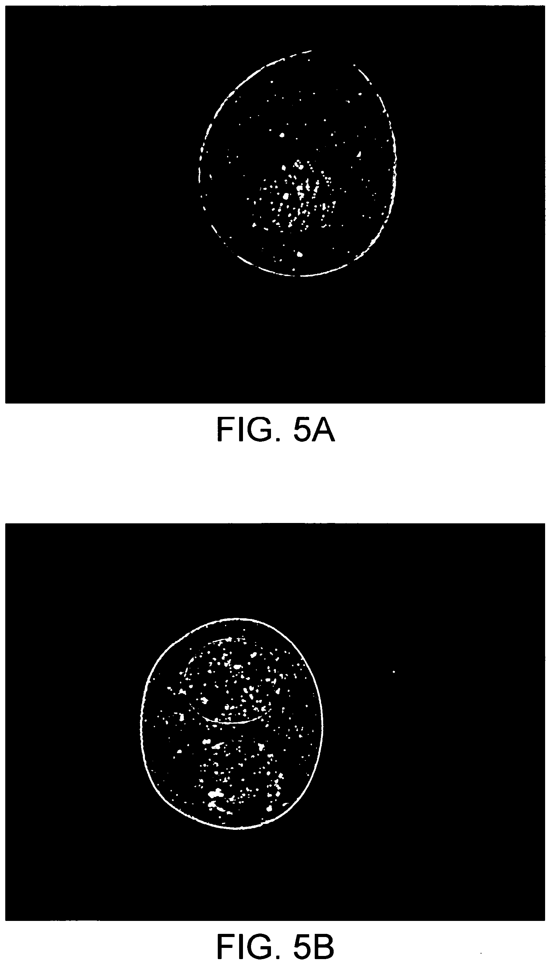

**Figures 5A** and **5B** are photomicrographs showing a fluorescent microcapsule encapsulated within a second microcapsule, and two fluorescent microcapsules encapsulated within a further microcapsule respectively.

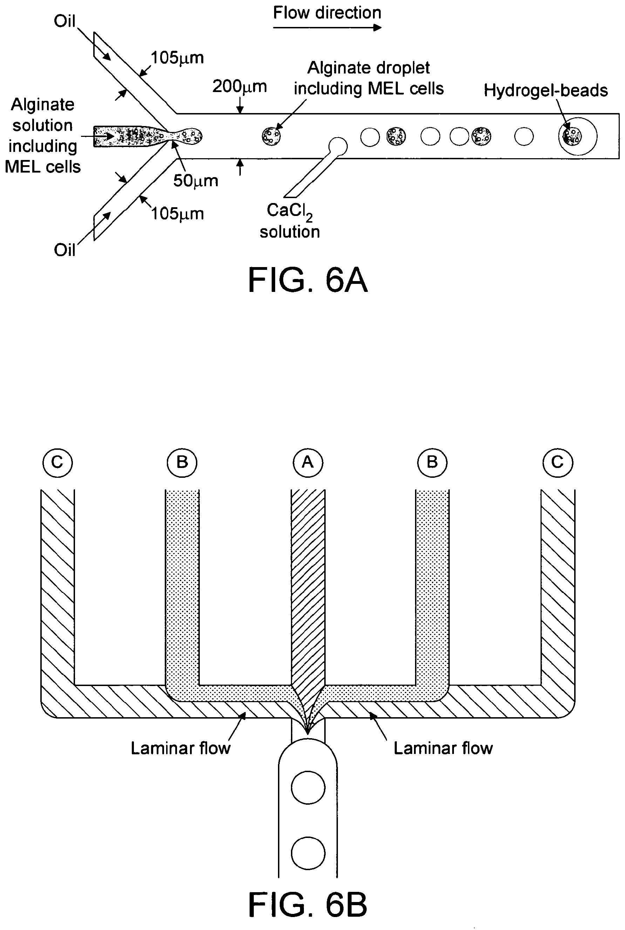

**Figures 6A** and **6B** are schematic of the formation of microcapsules by two microfluidic methods.

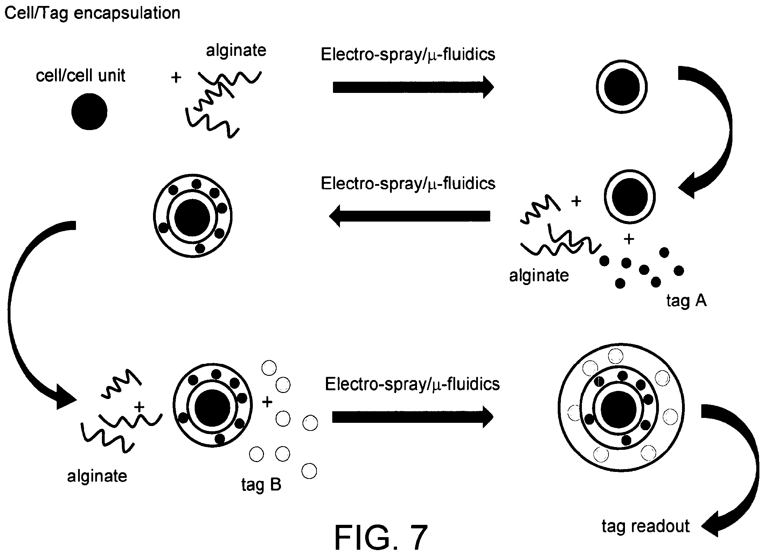

**Figure 7** is a general schematic of the microencapsulation of cell units and labels in microcapsules.

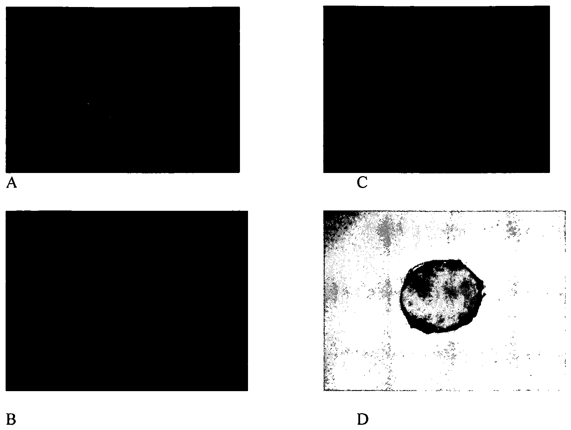

**Figures 8A-8D** are photomicrographs showing tags encapsulated within two layered spheres. Figure 8A shows tags fluorescing blue, whilst 8B shows tags fluorescing red. A combined image is shown in 8C. 8D is a brightfield photomicrograph. The red fluorescent image was recorded using a Cy5.5 optical filter (Excitation = 650/45 nm, Emission = 710/50 nm). The blue fluorescent image was recorded using a DAPI optical filter (Excitation = 360/40 nm, Emission = 460/50 nm).

### Detailed Description of the Invention

### Definitions

As used herein, the term "culture conditions" refers to the environment which cells are placed in or are exposed to in order to promote growth or differentiation of said cells. Thus, the term refers to the medium, temperature, atmospheric conditions, substrate, stirring conditions and the like which may affect the growth and/or differentiation of cells. More particularly, the term refers to specific agents which may be incorporated into culture media and which may influence the growth and/or differentiation of cells.

A "cell", as referred to herein, is defined as the smallest structural unit of an organism that is capable of independent functioning, or a single-celled organism, consisting of one or more nuclei, cytoplasm, and various organelles, all surrounded by a semipermeable cell membrane or cell wall. The cell may be prokaryotic, eukaryotic, animal or plant, or archaebacterial.

For example, the cell may be a eukaryotic cell. Mammalian cells are preferred, especially human cells. Cells may be natural or modified, such as by genetic manipulation or passaging in culture, to achieve desired properties. A stem cell is defined in more detail below, and is a totipotent, pluripotent or multipotent cell capable of giving rise to more than one differentiated cell type. Stem cells may be differentiated in vitro to give rise to differentiated cells, which may themselves be multipotent, or may be terminally differentiated. Cells differentiated in vitro are cells which have been created artificially by exposing stem cells to one or more agents which promote cell differentiation.

A "cell unit" is a group of cells, which may be a group of one. Groups or pools of cell units may be sorted, subdivided and handled without substantially dissociating the cell units themselves, such that the cell unit behaves as a colony of cells and each cell in the cell unit is exposed to the same culture conditions. For example, a cell unit may comprise a bead to which is adhered a group of cells, or a cell aggregate such as an islet structure or embryoid body, or a collection of non-adherent cells such as certain blood cells.

A "group" of cell units (or cells) is a plurality of such units which are not linked together. For example, a cell unit is not a group of cells, but one or more cells clustered together in one single unit. Single cells, and individual cell units, may be pooled to form a group of cells or cell units. Groups can be split, by dividing the groups into two or more groups of cells or cell units.

A "totipotent" cell is a cell with the potential to differentiate into any type of somatic or germ cell found in the organism. Thus, any desired cell may be derived, by some means, from a totipotent cell.

A "pluripotent" cell is a cell which may differentiate into more than one, but not all, cell types.

A "label" or "tag", as used herein, is a means to identify a cell unit and/or determine a culture condition, or a sequence of culture conditions, to which the cell unit has been exposed. Thus, a label may be a group of labels, each added at a specific culturing step; or a label added at the beginning or the experiment which is modified according to, or tracked during, the culturing steps to which the cell unit is exposed; or simply a positional reference, which allows the culturing steps used to be deduced. A label or tag may also be a device that reports or records the location or the identity of a cell unit at any one time, or assigns a unique identifier to the cell unit. Examples of labels or tags are molecules of unique sequence, structure or mass; or fluorescent molecules or objects such as beads; or radiofrequency and other transponders; or objects with unique markings or shapes.

An "identifying label" is a label which permits the nature of the cell unit to which it is attached to be determined. This allows the exposure of cell units to different culture conditions to be recorded, by addition of an identifying label at each exposure, and subsequently deconvoluted by analysis of the labels.

A cell is "exposed to culture conditions" when it is placed in contact with a medium, or grown under conditions which affect one or more cellular process(es) such as the growth, differentiation, or metabolic state of the cell.

Thus, if the culture conditions comprise culturing the cell in a medium, the cell is placed in the medium for a sufficient period of time for it to have an effect. Likewise, if the conditions are temperature conditions, the cells are cultured at the desired temperature.

The "pooling" of one or more groups of cell units involves the admixture of the groups to create a single group or pool which comprises cell units of more than one background, that is, that have been exposed to more than one different sets of culture conditions. A pool may be subdivided further into groups, either randomly or non-randomly ; such groups are not themselves "pools" for the present purposes, but may themselves be pooled by combination, for example after exposure to different sets of culture conditions.

"Cell growth" and "cell proliferation" are used interchangeably herein to denote multiplication of cell numbers without differentiation into different cell types or lineages. In other words, the terms denote increase of viable cell numbers. Preferably proliferation is not accompanied by appreciable changes in phenotype or genotype.

"Cell differentiation" is the development, from a cell type, of a different cell type. For example, a bipotent, pluripotent or totipotent cell may differentiate into a neural cell. Differentiation may be accompanied by proliferation, or may be independent thereof. The term 'differentiation' generally refers to the acquisition of a phenotype of a mature cell type from a less developmentally defined cell type, e. g. a neuron, or a lymphocyte, but does not preclude transdifferentiation, whereby one mature cell type may convert to another mature cell type e. g. a neuron to a lymphocyte.

The "differentiation state" of a cell is the level to which a cell has differentiated along a particular pathway or lineage.

A "microcapsule" is a protective barrier which encloses a cell unit. The term is commonly used in the art to refer to semi-permeable or impermeable structures; in the context of the present invention, microcapsules are semi-permeable and allow the passage of the components of growth media and other reagents, but retain labels and tags in order to allow identification of the cell units. Microencapsulation, or encapsulation, is the enclosing of a cell unit in a microcapsule.

When referring to distributions of numbers of cell units within microcapsules, or microcapsules within microcapsules, "substantially" is used to mean that the majority of the described distribution confers with the stated parameter; thus, if substantially each microcapsule contains a single cell unit, it is expected that most of the microcapsules obtained will comprise a single cell unit. Some may comprise two, three or more; however, these will be comparatively rare. Microcapsules with no cell units enclosed are not considered to be part of the distribution. The term "plurality" means more than one. In the context of labels, it describes the fact that each encapsulation allows at least one more label to be added, such that a multiply-encapsulated cell unit can be labelled with at least one label per encapsulation. In the context of cell units within microcapsules, two, three, four, five, six or more cell units can be included in a single microcapsule.

### Encapsulation

Encapsulation may be performed using any suitable technique. Microcapsule technology has been described, for example in US 6,808,882 and US 7,138,233 which describe emulsion microencapsulation technology in particular, but also set forth other microencapsulation methods at least some of which are suitable for encapsulating living cells.

Particular methods for encapsulating living cells have been described for immunoprotection of transplanted cells. For example, see Orive et al., (2203) Nature Medicine 9:104-107 and references cited therein.

One method suitable for encapsulating living cells is the layer-by-layer (LbL) addition of polyelectrolyte layers. In such a method, a cell is first encapsulated in a microcapsule, such as an alginate bead, and subsequently layers of alternately charged polyelectrolytes are added. The labels can be embedded in the layers, or the layers themselves can be labelled.

Polyelectrolytes include, for example, polystyrene sulfonate, polyallylammine, poly diallylmethylammonium chloride, polyethyleneimine, polyacrylic acid, polyvinylsulfonate and Nafion.

Layers of polyelectrolyte are added by suspending the cell or other surface to be coated in a solution of polyelectrolyte which is of sufficient concentration to form a saturated layer on the said surface. If excess polyelectrolyte is used, it must be removed before adding further polyelectrolytes to avoid complex formation in solution.

If polyelectrolytes are added sequentially at the concentrations required to form complete layers the desired microcapsules can be formed rapidly, because the polyelectrolytes are adsorbed in few minutes and no washing cycles are needed. However, the risk of forming free polyelectrolyte complexes in solution and cell aggregates is increased. This risk can be reduced by using a highly diluted suspension of cells, or determining accurately the exact amount of polyelectrolyte needed for fully coating the cells.

Therefore, in one embodiment, an excess of polyelectrolyte is added to the cells. After adsorption of each polyelectrolyte layer, excess polyelectrolyte is removed from the solution before adding oppositely charged polyelectrolyte to the cells. The excess is removed by performing at least three washing cycles, for example with PBS. Separation of the cells from the solution can be carried out by centrifugation or, preferably, filtration, the particles are filtered from the excess polyelectrolyte through coarse filters designed to retain cellular material.

In one embodiment, the polyelectrolytes alginate and poly-L-lysine may be used. Alginate and PLL are charged negatively and positively charged respectively. Other polyelectrolytes which are useful form encapsulating cells include polystyrene sulfonate and polyallylamine hydrochloride. For example, see Table 2 of Peyratout and Daehne, (2004) Angew. Chem. Int. ed. 43:3762-3783, as well as Leung et al., (2009) J Biomed Mater Res A 88:226-37. Labels or tags may also be charged; for example a negatively charged label can be used with a positively charged polyelectrolyte.

In one embodiment, cells may be coated with alginate as described herein or in the art, and subsequently washed and suspended in a solution comprising charged labels. Alginate is negatively charged, so a positively charged label will associated with the alginate.

Alternatively, or in addition, the cells can be suspended in a solution comprising a positively charged polyelectrolyte, such as poly-L-lysine (PLL), which may itself be labelled, or suspended in a solution of a polyelectrolyte such as PLL together with a positively or negatively charged label, which will associate with the alginate or the PLL respectively. Changing the time of suspension affects the thickness of the layer; times of from 1 minute up to 1 hour are possible, but times of 5 to 10 minutes are more common, using 0.05-0.1 wt% PLL.

Resulting beads are washed, for example in PBS, and then suspended in Alginate, which is optionally labelled. Again, typically a 0.05-0.1 wt% solution is used, for 5-10 miutes.

After encapsulation, beads are washed and then resuspended as desired to add further coats.

In one embodiment, the polyelectrolyte layers themselves may be detectable. For example, fluorescent or colour-coded polyelectrolytes may be used. See Strand et al., Biotechnol Bioeng (2003) 82:386-94.

In a further embodiment, encapsulation can be performed under microfluidic control. The formation droplets containing cells in microfluidic systems has been widely demonstrated and has been used for high throughput droplet based assays (J. Clausell-Tormos, et al., Chemistry and Biology, 2008, 15, 5, 427) and cell sorting (J-C. Baret, et al., Lab Chip, 2009, 9, 1850). In these examples cells are encapsulated within water droplets separated by an oil phase. The water droplets are stabilised by a surfactant layer.

The formation of hydrogel encapsulated cells has moreover been demonstrated using several methods. These include the formation of both alginate/cell water droplets and CaCl₂ containing water droplets within an oil phase. When the two types of droplets fuse together, a cell-containing cross-linked hydrogel bead is formed as shown in figure 1 of H. Shintaku, et al., Microsystems Technology, 2007, 13, 951, reproduced here as Figure 6A. This method is described more fully below.

There are two stages in the process; droplet formation, and the coalescence of droplets to form the hydrogel, as shown in Fig. 6A. For droplet formation, firstly a droplet of sodium alginate solution containing cells is formed from a nozzle located upstream of the microchannel using by introducing an aqueous phase into oil in a microchannel. The alginate droplet flows downstream in the main channel, following the flow of the continuous liquid phase. Secondly, the alginate droplet is fused with droplets of calcium chloride solution formed from a second nozzle located downstream.

In order to produce hydrogel beads smaller than 300 µm in their diameter, the channel depth is preferably about 50 µm, with a preferred diameter of 50 µm for the nozzle and 200 µm for the main channel, respectively.

Sodium alginate solution is preferably employed at a concentration of 1.5% by weight, and cells dispersed in the alginate at a concentration of 10⁵ cells/ml. Calcium chloride is preferably provided at a concentration of 0.1M. Vegetable oil such as sunflower oil can be used as the oil phase.

A second protocol has been described by Workman, et al., Macromolecular Rapid Communications, 2008, 29, 165). In this method, a shielded junction is employed to generate alginate microspheres (see Figure 1 in Workman *et al,* reproduced here as Figure 6B). Aqueous sodium alginate mixed with CaCO₃ and cells is introduced into a central channel. Sunflower oil mixed with acetic acid is supplied to the outermost channels (A). Sunflower oil is supplied to the intermediate channels (B) to act as a shield preventing the alginate solution from coming into contact with the acidified oil flow. Between channels B and A the two oils flow in a laminar fashion, with minimal diffusion of H⁺ into the protective sunflower oil. After droplet formation at the junction, H⁺ diffuses into the alginate droplet, thus liberating Ca²⁺ from CaCO3, which causes gelation of the alginate. Channels prior to the junction preferably have a cross-sectional area of about 500 µm², after the junction channels preferably about 1000 µm².

Encapsulation can moreover be performed using a jetting encapsulation technique. Many such techniques are known in the art; preferred are bio-electrospray jetting, aerodynamically-assisted bio-jetting and pressure-assisted cell jetting.

Electrospraying is also known as bio-electrospraying or electrohydrodynamic jetting, and relies on a potential difference between a spray nozzle or needle and a grounded electrode to produce droplets of defined size.

The media are passed through a conducting needle that is held at a higher potential than the electrode, setting up an external electric field into which the media exiting the needle are passed. Needles are hollow, having an internal diameter of between 0.2 and 2mm, and either flat or chamfered edge geometries. Needles may also be coaxial, such that different fluids can be sprayed from the same needle contemporaneously. The formation of the droplets is determined by the potential difference (difference in voltage) between the needle and the electrode, the flow rate of the medium and its relative features such as viscosity, surface tension, electrical conductivity and relative permittivity. Voltage and distance are related as the electric field depends on both variables. Normally, encapsulations are done at 1 or 2cm distance with voltages around 5-10kV. When the jet is stable, near monodistributions of droplet sizes can be achieved. Living cells can be encapsulated using this technology (Jayasinghe et al., (2006) Small 2, 216-219; and (2006) Biotechnol. J. 1:86-94). Although early experiments resulted in unstable jets with a wide dispersion of droplet sizes, this was improved using a coaxial jetting needle to create stable jetting (Jayasinghe et al., (2006) Lab Chip 6:1086-1090) with the microencapsulation material sprayed in the outer jet and the biomaterial in the inner jet. Aerodynamically assisted jetting relies on a pressure gradient. A pressure is created in a chamber, with respect to the surrounding atmosphere, which provides the drawing effect to create the jet. Living cells can be encapsulated in this way (Arumuganathar et al., (2007) Biomed. Mat 2:158-168).

Pressure-assisted jetting employs a coaxial needle, where one orifice is used to jet the medium, and the second serves as the conduit for a pressure to be applied. Unlike aerodynamically-assisted jetting, there is no pressurised chamber.

For a general review of jetting technologies, see Jayasinghe, S., (2008) Regen. Med. 3:49-61, as well as US 6,649,384, US 2006/0051329 and US 4,353,888.

The cells to be encapsulated may be naked individual cells, or may be incorporated into microcarriers, such as spheres or porous microcarriers, such as described herein.

In order to prepare the cell units for encapsulation, in one embodiment the cell units, which can be individual cells, are washed in a suitable aqueous buffer such as PBS, precipitated and resuspended in a buffer comprising the encapsulating polymer.

Encapsulation materials can be any suitable polymeric materials. The original encapsulation techniques used poly-dimethylsiloxane as the encapsulating material. Preferred are hydrogels, especially alginate. The polymer should be capable of ready solidification to form a membrane having the desired properties, and be insoluble in water or saline at physiological pH. The desired properties include nutrient permeability *in vivo,* and typically require that the polymer have a degree of polarity. The polymer membrane can typically contain 20-90% water at equilibrium. The polymer should be non-toxic to cells in solution. Examples of suitable polymers include polyacrylates and copolymers with acrylic acid, methacrylic acid and esters thereof, cellulose based polymers, copolymers containing acrylamides, N-vinyl pyrrolidone, styrene sulphonate, vinyl pyridine, vinyl alcohol, allyl alcohol and the like. A suitable polymer is a copolymer of acrylic acid ester and methacrylic acid ester, with small amounts of quaternary ammonium groups. See also US 6,281,241; and Desai, 2002 Exp. Opin. Biol. Ther. 2:633-646. Generally, polymers useful for encapsulating cells for immuno-protection purposes, as known in the art, are useful in the present invention. For example, see Orive et al., (2003) Nature Medicine 9:104-107 and references cited therein.

The encapsulating polymer is preferably a PBS buffer lacking calcium ions and magnesium ions (this prevents premature solidification of the encapsulating polymer). Typically, the buffer comprises 1-5% by weight of Alginate. Approximately 20ml of a 3% alginate solution in PBS is required to encapsulate 1g of microcarrier-based cell units.

Labels or tags may be added to the encapsulating polymer solution, or to the cell units before or after precipitation from PBS.

Gelling agents for the polymers can be introduced in one of three manners: (1) a secondary droplet population is generated and induced to fuse with the cell capsules; (2) the cell droplets are extracted into a water phase stream containing the polymerization agent established parallel to the oil phase; or (3) gelling agents are dissolved directly into the oil phase.

Preferably, Ca²⁺, Sr²+ or Ba²⁺ ions are used to solidify the alginate. CaCl₂, or the respective Sr or Ba compounds, are dissolved in water at a concentration of between 10mM and 1M. Combinations of Ca, Sr and Ba can be used with as little as 1mM of one ingredient to achieve optimum bead properties. This solution is preferably held in a collection vessel, which is placed at the electrode of an electrospray unit. The cell units, suspended in alginate solution, are passed through the spraying machine such that droplets are collected in the vessel which holds the solidifying or gelling solution. Encapsulated beads can be retrieved from the bottom of the vessel after spraying.

For re-encapsulation, the cell units are already encapsulated at the start of the procedure. The encapsulated cell units are advantageously washed in PBS and precipitated. 5ml of an alginate solution prepared as above, preferably at a concentration of about 2% by weight, is used for every 1ml volume of cell units with the liquid removed, as above. A label or tag may also be added. The units are jetted into a vessel containing a gelling agent, and re-encapsulated units collected at the bottom of the vessel. Advantageously, a slightly larger needle diameter is used, for instance 0.9mm, to compensate for the increased size of the biomaterial. In some embodiments, a higher flow rate and lower field strength can be used than in the primary encapsulation procedure.

In an alternative configuration, a coaxial spray needle can be used, with a cell suspension jetted in the inner needle and the encapsulating polymer solution in the outer needle. This arrangement has been shown to be capable of achieving a more stable jetting to encapsulate living cells (Jayasinghe & Townsend-Nicholson, (2006) Lab Chip 6:1086-1090).

### Tags or labels

As described above, tags may be used as labels. In methods of the prior art, tags have been conjugated to microcarriers or cell units, and this approach may apply in the first of the nested encapsulations according to the present invention, where the tags can be in direct contact with the cell units. In subsequent encapsulations, however, the tags are not in contact with the microcarriers in the cell units, and thus do not form complexes therewith.

Various molecular or macromolecular tags may be used. The tags typically comprise uniquely shaped objects, or objects modified with markings and/or coloured and/or fluorescent compounds.

In one embodiment the tags have one or more (preferably all) of the following qualities:
i. They are small in size relative to the cell unit they are labelling;
ii. They are separable from cell units under conditions which do not perturb the unique qualities of the tags; where the tags are not in direct contact with the cell units, for example when they are in an outer microcapsule layer, this feature may be more readily achieved by a wider variety of tag chemistries;
iii. They are made of one or more inert substances which do not substantially affect the biology of the cell unit and which in turn is not affected by cell units or their biology; again, this may be more readily achievable with a wider variety of tags in the outer microcapsule layers;
iv. They are obtainable in large numbers and moreover in many related but distinct variants which are easily distinguishable using an appropriate technique;
v. They are distinguishable by a method which is convenient, highly reliable and which can be automated, for example by FACS.

In one embodiment, the tag is a microsphere, such as a fluorescent and/or coloured microsphere. More than 2000 different microspheres are available, made by emulsion or suspension polymerization, precipitation etc. and comprised of polystyrene, other polymers, copolymers, terpolymers and/or silica etc. Microspheres are manufactured in a variety of sizes, densities, colours etc, for example by Bangs Laboratories Inc., (Fishers IN, USA).

A common type of microsphere is the Polystyrene (PS) and styrene/divinylbenzene copolymer (S/DVB) microsphere. Other polymers include polymethylmethacrylate (PMMA), polyvinyltoluene (PVT), styrene/butadiene (S/B) copolymer, and styrene/vinyltoluene (S/VT) copolymer. Suitably, the microsphere is a hydrophilic microsphere. More suitably the microsphere is a polystyrene microsphere. Most suitably, the microsphere is a surface-modified microsphere such as a carboxylate-modified microsphere.

Many of these microspheres can be functionalised, for instance by carboxyl group as in the CML microspheres, or by amino functionalized or nitrogen-containing compounds, like primary, secondary, tertiary, and quaternary aliphatic amines, aromatic amines, and pyridines, which offer alternative coupling reactions to the COOH beads.

CML microspheres have a highly charged surface layer of carboxyl groups derived from a copolymerisation process. The surface is somewhat porous and relatively hydrophilic, but retains overall hydrophobic properties. The charge density of these particles ranges from about 10-125 Å² per carboxyl group and they are stable to high concentrations -of electrolytes (up to -1M univalent salt). The CML latex, will adsorb proteins and other biomolecules, but much less strongly than hydrophobic microspheres.

In some embodiments, conjugates of microspheres and proteins, e.g. streptavidin, are prepared.

For example, conjugates with CML microspheres may be prepared as follows. CML microspheres may be activated using a water soluble carbodiimide reagent that makes the carboxyl groups reactive with primary amines on the proteins to be coupled. A 50 mM reaction buffer at pH 6.0 is prepared. Sodium acetate or 2-(N- morpholino) ethanesulfonic acid (MES) are suitable buffers. The protein is dissolved in the reaction buffer at a concentration of 10 mg/mL. A 1% (w/v) suspension of microspheres is prepared in the reaction buffer. One volume protein solution to ten volumes microsphere suspension is prepared and the mixture allowed to incubate, at room temperature for 20 minutes. A solution of 10 mg/mL (52 µMοl/mL) of 1-ethyl-3-(3-dimethylaminopropyl) carbodiimide (EDAC) in deionized water is prepared and used immediately. A calculated amount of EDAC solution to the microsphere suspension is added and the pH of the reaction mixture adjusted to 6.5 ± 0.2 with 0.1N NaOH. The mixture is incubated on a rocker or mixing wheel for 2 hours at room temperature. Unbound protein is removed and stored in storage buffer.

Advantageously, CML and other microspheres can be obtained in various formats, such as various colours (e.g. blue, red, green, yellow, black), various fluorophores (eg. Fluorescein (green), Fluorescein (red) or Fluorescein and Rhodamine (red and green) and various sizes (e.g. 5.4 µm (1.14 x 10¹⁰ beads/gram), and 7.6 µm (4.10 x 10⁹ beads/gram)).

CML-and other microspheres may be prepared such that they are loaded with one or more visible dyes and/or fluorophores.

In some embodiments, tags, such as microspheres, are not coated with proteins.. By varying various parameters in the fabrication process, commercial microsphere providers - such as Bangs Laboratories - can manufacture bead sets which can be distinguished based on differing sizes (e.g. bead sets of 4.4µm and 5.5µm diameter). Beads within each size group can be furthermore distinguished from each other based on differing fluorescence intensity owing to differential loading with a single fluorescent dye. It is possible to use many different dyes with different absorption or emission characteristics, which can be attached to the microcarriers described herein. Accordingly, Tag diversity may result from varying tag size and/or fluorophore loading (the fluor intensity) and/or fluorophore identity/combination. In particular, Tag diversity may result from the type of fluorophore they carry (eg. beads can be loaded with either UV2 5 or Starfire Red); size (eg. for each fluorophore there are 5 different bead sizes: 1.87, 4.41, 5.78, 5.37 and 9.77 microns) and/or the quantity of fluorophore they carry (5 different intensities of each dye are available). Other fluorophores - such as TRITC may be used. Filters can then be used to detect the at least 4 different dyes on any given bead, such as the TRITC filter (ex 540/25; dm 565; ba 605/55) for TRITC visualization from Nikon; the DAPI filter (ex 340-380; dm 400; ba 435-485) for UV2 visualization from Nikon; the GFP-B filter (ex 460-500; dm 505; ba 510-560) for FITC visualization from Nikon and the Cy5 filter set (cat- no 4t008 from Chroma Technology) for Strarfire Red visualization.

Microspheres can be dyed internally or externally, with visible or fluorescent dyes. Internal dyeing occurs when the dye is integrated into the microsphere mass, typically by-soaking the microsphere in a solution containing a dye or fluorophore. External modification occurs when a dye is conjugated to the surface of the microsphere, for instance modification of a CML microsphere with an isothiocyanate derivative as described herein.

Accordingly, in some embodiments, the microsphere may be dyed internally or externally, with visible or fluorescent dyes.

It is furthermore possible to use 'quantum dots' to obtain a very high number of different fluorescent labels which can be read conveniently. Thus in another embodiment of the present invention, quantum dots are used instead of fluorophores. In certain embodiments, quantum dots are preferable due to the fact they do not fade (photo-bleach) when exposed to light. For instance the fluorophore FITC is known to photo-bleach and cell units treated with tags containing FITC are ideally handled in the dark and are difficult to analyse reliably. Quantum dots may be incorporated into microspheres at the time of polymerizing the polystyrene resulting in even loading of tags. Quantum dots are available in many colours and they can be excited at the same wavelength so allowing visualization of multiple colours without filters, by using a colour CCD camera. Further background information on Quantum dots is available from US 6,322,901, US 6,576,291, US 2003/0017264, US 6.423,551, US 6,251,303, US 6,319,426, US 6,426,513, US 6,444,143, US 2002/0045045, US 5,990,479, US 6,207,392, US 6,251,303, US 6,319,426, US 6,426,513 and US 6,444,143. Detection of tags can be accomplished by a variety of methods familiar to those skilled in the art. Methods include mass spectrometry, nuclear magnetic resonance, sequencing, hybridisation, antigen detection, electrophoresis, spectroscopy, microscopy, image analysis, fluorescence detection, and the like. In some embodiments, since the tags typically contain a colour or a fluorophore then microscopy, spectroscopy, image analysis and/or fluorescence detection are used.

Detection of tags can be performed in situ, that is to say without perturbing the structure of the encapsulated cells, or may be performed after isolating tags from the capsule.

An advantage of detecting tags in situ is that it is possible to take advantage of combinatorial tagging strategies. For example when using tags A, B and C it would be possible to create different encapsulation layers that contain the unique tag combinations AB, AC and BC, in addition to layers that contain only tags A, B and C. In one embodiment the layers also contain a second easily detected differentiator, such as a dye, which additionally allows the different layers to be discriminated.

In one embodiment encapsulation of tags can result in a spatially encoded polymer matrix; for example, see United States Patent Application 20060127369.

Multiple variations of a tagging strategy can be devised to determine the identity of a given cell unit in a mixture or of deducing the identity of the different cell units that are comprised in a mixture. For instance optical or visual methods of tagging have been described where different shaped objects, graphically encoded objects or different colours denote the identity of a sample (for example see, Guiles et al, 1998, Angew. Chem. Intl Ed Engl, vol. 37, p926; Luminex Corp, Austin TX, USA; BCPbiosciences; Memobead Technologies, Ghent, Belgium).

In a further embodiment, the tag is a rod-shaped particle. Suitably, the rod-shaped tag is a nanowire. The nanowire may comprise, consist or consist essentially of various metals, such as aluminium. The nanowire may be coated with various metals, such as silver and/or gold. Suitably, the nanowire is about 1µM or less in diameter and/or is about 10 µM or less in length.

The nanowire may be a nanowire as described in Science, vol 294, p. 137-141 (2001). Briefly, nanowires are multimetal microrods intrinsically encoded with submicrometer stripes. Complex patterns can be generated by sequential electrochemical deposition of metal ions onto templates with uniformly sized pores. Advantageously, the nanowires are small enough to be used as tags that may be added after each split.

Parameters for the rod-shaped particle, such as the nanowire, include but are not limited to size, optical properties and/or metal composition. In one embodiment, the optical properties are selected from the group consisting of: light reflectivity, such as light reflectivity of a particular wavelength, colour, the fluorescence emission-wavelengths and the fluorescence emission intensity.

In some embodiments, the rod-shaped particle, such as the nanowire, is externally dyed.

Rod shaped tags may be preferable to spherical tags since the higher surface area to volume ratio results in superior retention in the microcapsule layers. Accordingly, the binding of nanowires can be better than, for example, the binding of microsphere tags and results in a high level tagging.

For some embodiments, the tag is not a DNA tag.

In some embodiments, the tag is an externally dyed tag.

In a further embodiment the tags use radio waves to transmit information, as in RFID tags. RFID generally employs transponders (RF tags), antennae and readers. An RF tag is a small electronic circuit, usually encased in glass or plastic, which in its simplest form provides access to a unique identification code that may be 'read', without contact or line of sight, by suitable electronics. Tags may also store information generated by the user, again without contact or line of sight. A 'reader' is an electronic unit that transfers information to and from one or more tags (it should be noted that the term reader is used interchangeably to mean both a read only and read/write unit). The size and features of a reader may vary considerably, and it may operate in isolation, or be connected to a remote computer system. An antenna is used to transmit information from a reader to a tag, and to receive information sent by an RF tag. The size and format of an antenna will reflect the specific application, and may range from a small circular coil to large planar structures. An RFID system may operate in isolation, or be connected to a remote computer for more comprehensive interpretation and manipulation of identification and associated data derived from a tag. One RFID strategy is described in Nicolaou et al (1995, Angew Chem Intl Ed Engl, vol. 34, p. 2289) and comprises: (i) a porous enclosure containing a synthesis substrate and the semiconductor tag; (ii) the solid phase synthesis resin; (iii) a glass-encased Single or Multiple Addressable Radiofrequency Tag semiconductor unit capable of receiving, storing and emitting radiofrequency signals. A similar device could be adapted to growing and following cell units simply by replacing the solid phase synthesis resin with tissue culture microcarriers or suitable cell units. More variations of this can be envisaged including but not limited to (coated or uncoated) RF tags on which cells are grown directly, or RF tags implanted into cell units or organisms.

### Labelled Microcapsule Walls

In an alternative, or complementary, labelling strategy, label can be incorporated into the material from which the microcapsule wall is constructed. For example, fluorescently-labelled polymers can be used to coat the cells. If the polymer is alginate, for example, labels such as fluorescinamine (C₂₀H₁₃NO₃) can be reacted with the alginate by exposing carboxylic groups on the alginate. Poly-L-lysine (PLL) can be labelled using a commercial protein labelling reagent, such as the Alexa® 546 protein labelling kit from Molecular Probes (Leiden, Netherlands). For example, see Strand et al., Biotechnol Bioeng (2003) 82:386-94.

Fluorescent dyes can be used to label polyelectrolytes, such as those used in LbL encapsulation. See, for example, US2006/0105335, in which fluorescein isothiocyanate, tetramethylrhodamine isothiocyanate and a derivative of CY5 are used to label polyallylammine (PAH). In general, polyelectrolytes are labelled in accordance with general protocols for labelling proteins.

### Microcarriers

A variety of microcarriers are available, ranging in shape and size and made of different materials. Microcarriers may be porous, macroporous, microporous or solid. By way of example, the microcarrier may be a microcarrier selected from the group consisting of a Cultispher microcarrier, a Cultispher-G microcarrier, a Cultispher-GL microcarrier, a Cultispher-S microcarrier, an Informatrix microcarrier, a Microsphere microcarrier, a Siran microcarrier, a FibraCel® Disks microcarrier, a Cytoline microcarrier (*e.g.* a Cytoline 1 microcarrier or a Cytoline 2 microcarrier), a Cytodex microcarrier (*e.g.* a Cytodex 1, Cytodex 2 or Cytodex 3 microcarrier), a Cytopore microcarrier (*e.g.* a Cytopore 1 microcarrier or a Cytopore 2 microcarrier), a Biosilon microcarrier, a Bioglass microcarrier, a FACT III microcarrier, a Collagen C microcarrier, a Hillex II microcarrier, a ProNectin F microcarrier, a Plastice microcarrier, a Plastic Plus microcarrier, a Nunc 2D MicroHex™ microcarrier, a Glass microcarrier (Sigma Aldrich), a DE 52/53 microcarrier or combinations thereof.

### Cells and cell units

Incorporation of cells into cell units leads to the formation of an entity that facilitates groups of cells (cell colonies) to be grown in cell culture under various conditions and the maintenance of the integrity of the group under various conditions, when disturbed and when mixed with other colonies. Such groups or colonies are referred to herein as cell units. Formation of cell units may be achieved, by way of illustration, by growing cells as adherent cultures on solid substrates, such as carriers. If cell proliferation occurs after seeding on the carriers, the daughter cells will attach on the same carrier, and form part of the same colony. In general, live adherent cells do not readily dissociate from their growth substrate, and so the integrity of the cell colony persists despite any mechanical manipulation of the carrier, agitation of the culture medium, or transfer into another tissue culture system. Similarly, if at any time multiple carriers are placed in the same vessel (e.g. beads are pooled) then there will be no substantial transfer of cells from one bead to another.

When cell units are formed on solid substrates, the substrate, and therefore the attached cells by reason of association, can be labelled as described herein. Cells grown smaller carriers can be treated as a suspension culture. A common method of growing cells on small carriers is referred to as microcarrier cell culture (see 'Microcarrier cell culture", Principles and Methods', Edition AA, available from Amersham Biosciences (18-1140-62)). Microcarrier cultures are used commercially for antibody and interferon production in fermenters of up to 4000 litres.

An advantage of encapsulating cell units is that this significantly reduces any transfer of cells (or tags) from one unit to another.

As the physical properties of carriers are well known it is easy to calculate the number of carriers used in an experiment. The carriers may be available as dried products, which can be accurately weighed, and subsequently prepared by swelling in liquid medium. In addition the number of cells used to inoculate a microcarrier culture can be worked out and varied.

Harvesting of cells grown on the microcarriers described herein, or liberation of labels from microcarriers when required, can be achieved by enzymatic detachment of cells, and/or by digestion of the carrier where applicable as described herein.

In some embodiments, the tag is a sphere, such as a microsphere, that comprises, consists or consists essentially of polystyrene.

The cells may be any cells, including differentiated cells, progenitor cells or stem cells, whether pluripotent or totipotent. In one embodiment, human embryonic stem cells are excluded. Types of cell that can be encapsulated are described, for example in WO2004/031369, WO2007/063316 and WO2007/023297.

### Kits

The invention moreover relates to kits for cell encapsulation. Such a kit comprises at least sodium alginate, one or more microcarriers and one or more detectable tags. The alginate may be in solid or solution form, and the solution may be ready to use or intended for dilution. Microcarriers and tags may be selected from those described above.

Kits may also include, for example, microfluidic nozzles or jetting needles suitable for encapsulation of cells, for instance as described herein, encapsulation polymers, such as polyelectrolytes or polymers suitable for jetting as described above, and gelling agents, which are typically calcium, strontium or barium ions.

Cells for encapsulation may moreover be included as part of a kit, as may media such as DMEM or PBS.

Specific kits may be designed for encapsulation by LbL, electrospray or microemulsion techniques. Each kit preferably comprises alginate, markers and microcarriers; however, polymers and media may differ from application to application. For example, a kit designed for electrospray encapsulation may comprise, in addition to alginate, tags and microcarriers, one or more components selected from polyacrylates and copolymers with acrylic acid, methacrylic acid and esters thereof, cellulose based polymers, copolymers containing acrylamides, N-vinyl pyrrolidone, styrene sulphonate, vinyl pyridine, vinyl alcohol, allyl alcohol and copolymers of acrylic acid ester and methacrylic acid ester, with small amounts of quaternary ammonium groups. The kit may include a gelling agent, which preferably comprises Ca²⁺, Sr²⁺ or Ba²⁺ ions. Also included may be one or more jetting needles, which advantageously has an internal diameter of between 0.2 and 2mm, and either flat or chamfered edge geometries.

Kits designed for LbL may comprise, for example, one or more polyelectrolyte solutions in addition to alginate, tags and microcarriers.

### Combinatorial serial culture of cells

### Split-pool cell culture

Forming cell units (particularly microscopic cell units) is furthermore useful for sampling multiple tissue culture conditions as each cell unit constitutes an easily handled unit that can be exposed to a variety of cell culture conditions. In accordance with the present invention, cell groupings are typically produced by growing cells in microcarrier culture, and the terms cell unit, cell group, colony and bead are used to describe components of the microcarrier cell culture. A particularly efficient method for sampling a large number of cell culture conditions is referred to as Combinatorial Cell Culture or split-pool cell culture and in one embodiment involves the serial subdividing and combining of groups of cell units in order to sample multiple combinations of cell culture conditions. In one aspect of the invention the method operates by taking an initial starter culture (or different starter cultures) of cell units divided into X1 number of aliquots, each containing multiple beads (groups/colonies/carriers) which are grown separately under different culture conditions. Following cell culture for a given time, the cell units can be encapsulated with a specific label, and can then be pooled by combining and mixing the beads from the different aliquots. This pool can be split again into X2 number of aliquots, each of which is cultured under different conditions for a period of time, encapsulated with another specific label (which can be the same or, preferably, different) and subsequently also pooled. This iterative procedure of splitting, culturing, encapsulating and pooling (or pooling, splitting and culturing; depending on where one enters the cycle) cell units allows systematic sampling of many different combinations of cell culture conditions. The complexity of the experiment, or in other words the number of different combinations of cell culture conditions tested, is equal to the product of the number of different conditions (XI x X2 x ...Xn) sampled at each round. Note that the step of pooling all the cell units prior to a subsequent split can be optional; a step in which a limited number of cell units are pooled can have the same effect. The invention therefore embodies a number of related methods of systematically sampling multiple combinations of cell culture conditions where groups of cell units are handled in bulk.

Following each round of cell culture, or after a defined number of rounds, the cell units can be assayed to determine whether there are members displaying increased cell proliferation or a particular phenotype or genotype. This can be achieved by a variety of techniques, for instance by visual inspection of the cell units under a microscope, or by quantitating a marker product characteristic of the cell. This may be an endogenous marker such as a particular DNA sequence, or a cell protein which can be detected by a ligand or antibody. Alternatively an exogenous marker, such as green fluorescent protein (GFP), can be introduced into the cell units being assayed to provide a specific readout of (living) cells in real time. Conversely, dead cells can be labelled using a variety of methods, for instance using propidium iodide. Furthermore the labelled cell units can be separated from unlabelled ones by a variety of techniques, both manual and automated, including fluorescence activated sorting, for instance using a COPAS instrument (Union Biometrica).

Further examples of split-pool (and split-split) culture protocols may be found in WO2004/031369, WO2007/063316 and WO2007/023297.

The invention is further described, for the purposes of illustration, in the following examples.

### Example 1

### Preparation of an alginate solution

Alginate in powder form is mixed into de-ionized water or phosphate buffered saline -CaCl₂ -MgCl₂ (PBS--), a PBS solution without Ca or Mg ions, to give a mixture by weight percentage of between 1-5% Alginate. About 20 ml of 3% dissolved in PBS per g of microcarriers is used. The mixture is left to stir until completely dissolved.

The solution is then autoclaved or sterile filtered to ensure that the solution is sterile.

### Preparation of a solidifying solution

Ca²⁺, Sr²⁺ or Ba²⁺ ions will crosslink the alginate solution into a hydrogel. A solidifying solution is made by dissolving CaCl₂ in water at a concentration of 200mM. The solution is stirred with a magnetic stirrer until dissolved completely.

### Preparation of the Encapsulator

Encapsulation is performed using a NISCO Encapsulator (NISCO Engineering AG, Switzerland). If not done before, the machine and all its components are cleaned and disinfected, using new silicone piping.

A needle having an internal diameter of 0.8mm and a flat (as opposed to chamfered) end geometry is selected, inserted into the luer lock of the spraying machine and tightened by hand.

The ground electrode is inserted into the assembly, the screws tightened and the ground electrode cables connected. Alternatively, the electrodes may be charged, and the needle grounded.

The distance of the needle tip to the electrode is set at 2cm, using a voltage of 7kV.

The CaCl₂ solution is placed into a small beaker comprising a magnetic stirring-rod, underneath the needle. The built-in magnetic stirrer is set at 30% and switched on at this point. The ground electrode rod from the assembly reaches into the liquid, thus grounding it, to prevent the liquid becoming charged and repelling the droplets.

### Preparation of the suspension

The units to be encapsulated, be it microcarriers, cells (clusters or dispersed) or beads from previous encapsulation steps, are washed in PBS, the liquid taken off and re-suspended in the alginate solution.

1ml of this suspension is taken into a syringe and injected directly into the piping of the encapsulator. By doing this, settling of the beads/microcarriers in the syringe is avoided.

### Preparation for Spraying

A second syringe is filled with plain alginate to push through the material to be encapsulated, and connected to the syringe pump.

The flow rate of the syringe pump is set at 5ml/h. Flow rates be in the range of about 1-20ml/h may be used.

### Spraying

In order to spray droplets, the voltage is applied across the needle and electrode are per the instructions for the machine. The procedure may be repeated, preparing more beads and injecting them into the machine, in order to encapsulate further material.

### Retrieval of beads

When processing has finished, the syringe pump, voltage and magnet stirrer are turned off and the collection beaker containing cross-linking solution and encapsulations is removed. Encapsulations settle on the bottom of the beaker and can be removed into a falcon tube or other vessel.

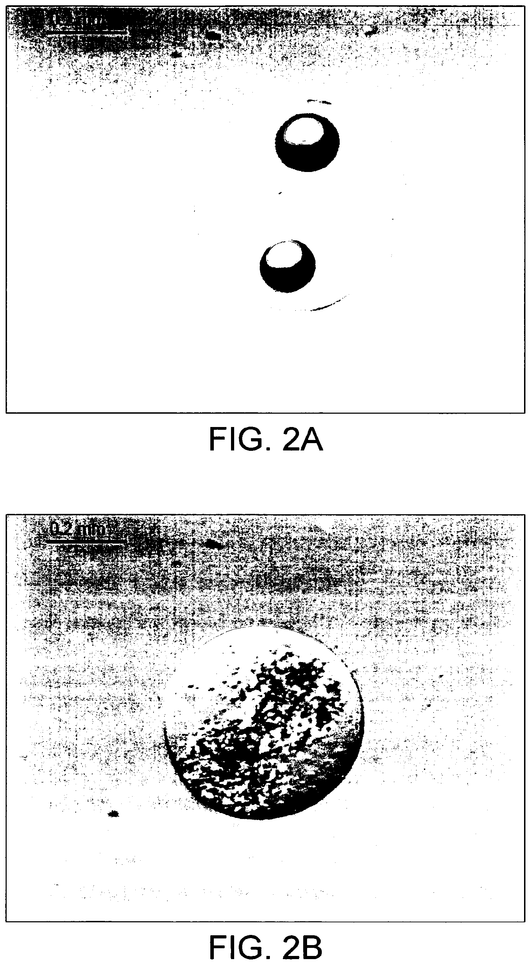

Beads are washed with PBS several times and re-suspended in the desired solution (PBS, DMEM, etc.). The results are shown in Figures 1 to 4.

### Machine Settings and variables

### Encapsulating microcarriers

| Suspension | 20 ml PBS-alginate, 2.5%, per 1 g of microcarriers |
|---|---|
| Needle | 0.8 mm flat end |
| Flowrate | 5 ml/h |
| Field strength | 7 kV |
| Electrode Distance | 2 cm |
| Cross-linker | CaCl₂, 200mM |

### Re-encapsulating existing encapsulations.

| Suspension | 5ml of 2% alginate (water based) per ∼1ml of washed beads, with liquid removed |
|---|---|
| Needle | 0.9 mm flat end |
| Flowrate | 10 ml/h |
| Field strength | 5 kV |
| Electrode Distance | 2 cm |
| Cross-linker | CaCl₂, 200mM |

### Example 2

Cell units are coated with alginate in accordance with Example 1, and subsequently coated with polyelectrolytes using a LbL approach as follows.

The polyelectrolytes, in this example alginate and PLL, are charged negatively and positively charged respectively. In essence, Example 1 provides the following four steps:
1. Prepare alginate suspension with cells
2. Prepare spraying equipment
3. Make basic alginate bead
4. Wash bead

The beads are then coated with PLL by suspending in a PLL solution. The charge difference attracts the PLL to the alginate. Optionally, a step can be included in which charged tags are attached to the alginate, to label the first encapsulation.
5. Suspend in a solution containing poly-1-lysine (PLL)
   a. A solution of 0.1% used for as little as 30s will already give the desired effect of creating an extremely thin film on the surface of the alginate bead. Changing the concentration or the time the beads are suspended in this solution will change the adsorption rate but the layer appears to be saturated and sufficient after no more than a minute at this concentration. The exact thickness is not known but small enough not to be visible on an optical microscope.
   b. Preferred settings are 1% for 30s. Settings may be varied between 0.05-0.1 wt% PLL in saline for less than a minute, up to several hours. Times of around 5-10 minutes are preferred.
6. Wash with PBS(- -)
   a. It is necessary to wash the beads several times in a cell strainer to remove excess PLL
7. Suspend with fluorescently labelled tags
   a. When tags are added and the solution agitated, most easily done by pipetting the liquid, the negatively charged tags attach to the surface of the Alginate-PLL bead.
8. Wash off excess tags with PBS(--)
   a. Again, done in a cell strainer (70µm) to retain the beads while washing away unattached tags.
9. Suspend beads in a prepared solution of another anionic polyelectrolyte.
   a. This can be done in alginate. Values such as 0.05-1% for 10 minutes appear common (e.g. (Strand et al., 2003)). However, the layers can become thick and uneven (it is possible to circumvent this by using different alginate or by modifying it [such as using a shorter chain length or a lower molecular weight])
   b. Use a solution containing poly(sodium styrene sulfonate) (PSS), with similar exposure times and concentrations, to form a second layer around the PLL.
   c. This second layer is to fix the tags and to add another layer of the opposite polarity onto which the first polyelectrolyte (a cationic substance) can be added again the following week to repeat the process with another set of tags.
10. Wash with PBS(- -) to remove excess poly-electrolyte
11. Suspend in cell medium until next split-pool stage of the experiment.
12. At the next split-pool stage where the beads are to be tagged, repeat steps 3-10 as necessary to add extra layers

Figure 8 shows an example of a bead after adding two layers with different fluorescent tags. First, the protocol was followed with blue fluorescent tags and subsequently steps 3-10 were repeated with red fluorescent tags to add a second layer containing tags.

The protocol was as follows:
Original alginate beads: 6.5 kV, 10 ml/h, 1cm distance, 0.5mm needle.
1^{st} Layer:
   PLL0.1% for 120sec
   Wash in cell strainer with approx. 25ml PBS--
   BLUE fluorescent tags
   Wash in cell strainer with approx. 10ml PBS--
   Alginate 0.5% for 120sec
   Wash in cell strainer with approx. 25ml PBS--
2^{nd} Layer:
   PLL0.1% for 120sec
   Wash in cell strainer with approx. 25ml PBS--
   RED fluorescent tags
   Wash in cell strainer with approx. 10ml PBS--
   Alginate 0.5% for 120sec
   Wash in cell strainer with approx. 25ml PBS--

Fig. 8A shows the result under a red filter only (Cy5.5 optical filter, Excitation = 650/45 nm, Emission = 710/50 nm). 8B shows the same bead under a blue filter (DAPI optical filter, Excitation = 360/40 nm, Emission = 460/50 nm). Figures 8C & D show a composite of the fluorescence and a bright field image of the tagged multi-layer bead respectively.

## Claims

1. A method for identifying a cell unit and/or determining a culture condition, comprising the steps of:
(a) providing one or more cell units each comprising one or more cells and exposing the cell units to desired culture conditions;
(b) microencapsulating the cell unit(s) obtained in step (a) together with one or more first labels;
(c) exposing the microencapsulated cell unit(s) obtained in step (b) to desired culture conditions;
(d) repeating the microencapsulation of step (b) together with a second label; and
(e) detecting the first label and the second label,
wherein the first and second labels are different.

2. A method for identifying a cell unit and/or determining a culture condition, comprising the steps of:
(a) providing a first group of one or more cell units each comprising one or more cells and exposing the cell units to desired culture conditions;
(b) microencapsulating the cell units obtained in step (a) together with a first identifying label;
(c) mixing the microencapsulated cell units obtained in step (b) with one or more cell units which have been exposed to different culture conditions and microencapsulated with a second identifying label to form a second group;
(d) repeating step (b) with the second group, using a third identifying label;
(e) detecting the first, second, and third identifying labels,
wherein at least two of the first, second, and third identifying labels are different.

3. The method according to claim 2, wherein the first group of cell units is split into two or more groups before or after step (c).

4. A method for identifying a cell unit and/or determining a culture condition, comprising the steps of:
(a) providing a first set of groups of cell units each comprising one or more cells, and exposing said groups to desired culture conditions;
(b) microencapsulating the cell units obtained in step (a) together with a first identifying label;
(c) pooling two or more groups obtained by step (b) to form at least one pool;
(d) subdividing the pool obtained in step (c) to create a further set of groups of cell units;
(e) exposing the further groups of cell units obtained in step (d) to different culture conditions;
(f) repeating steps (b) - (d) with a second identifying label;
(g) detecting the first and second identifying labels,
wherein the first and second labels are different.

5. The method according to claim 2 or claim 3, wherein the microencapsulation is performed by a jetting procedure, which is optionally selected from the group consisting of bio-electrospray jetting, aerodynamically-assisted bio-jetting and pressure-assisted cell jetting.

6. The method according to any one of claims 2 to 4, wherein the microencapsulation is performed by layer-by-layer addition of polyelectrolytes.

7. The method according to any one of claims 2 to 6, wherein the cell units comprise at least one microcarrier.

8. The method according to claim 2 or claim 3, wherein the culture conditions are selected from the group consisting of growth media, temperature regimes, substrates, atmospheric conditions and physical cell handling.

9. The method according to any one of claims 1 to 8, wherein substantially each microcapsule contains a single cell unit.

10. The method according to any one of claims 1 to 8, wherein substantially each microcapsule contains a plurality of cell units.

11. The method according to any one of claims 1 to 8, wherein each microcapsule contains a plurality of cell units, each cell unit itself being individually microencapsulated.

## Patentansprüche

1. Verfahren zum Identifizieren einer Zelleinheit und/oder Bestimmen einer Kulturbedingung, umfassend die Schritte:
(a) Bereitstellen einer oder mehrerer Zelleinheiten, wovon jede eine oder mehrere Zellen umfasst, und Aussetzen der Zelleinheiten gewünschten Zellkulturbedingungen:
(b) Mikroverkapseln der in Schritt (a) erhaltenden Zelleinheit(en) zusammen mit einem oder mehreren ersten Markern;
(c) Aussetzen der in Schritt (b) erhaltenen mikroverkapselten Zelleinheit(en) gewünschten Zellkulturbedingungen;
(d) Wiederholen der Mikroverkapselung von Schritt (b) zusammen mit einem zweiten Marker; und
(e) Nachweisen des ersten Markers und des zweiten Markers,
wobei die ersten und zweiten Marker verschieden sind.

2. Verfahren zum Identifizieren einer Zelleinheit und/oder Bestimmen einer Kulturbedingung, umfassend die Schritte:
(a) Bereitstellen einer ersten Gruppe einer oder mehrerer Zelleinheiten, die eine oder mehrere Zellen umfassen, und Aussetzen der Zelleinheiten gewünschten Zellkulturbedingungen;
(b) Mikroverkapseln der in Schritt (a) erhaltenen Zelleinheiten zusammen mit einem ersten Identifizierungsmarker;
(c) Mischen der in Schritt (b) erhaltenen mikroverkapselten Zelleinheiten mit einer oder mehreren Zelleinheiten, die unterschiedlichen Zellkulturbedingungen ausgesetzt und mit einem zweiten Identifizierungsmarker mikroverkapselt wurden, um eine zweite Gruppe zu bilden;
(d) Wiederholen von Schritt (b) mit der zweiten Gruppe unter Verwendung eines dritten Identifizierungsmarkers;
(e) Nachweisen der ersten, zweiten und dritten Identifizierungsmarker,
wobei wenigstens zwei der ersten, zweiten und dritten Identifizierungsmarker verschieden sind.

3. Verfahren nach Anspruch 2, wobei die erste Gruppe von Zelleinheiten vor oder nach Schritt (c) in zwei oder mehr Gruppen geteilt wird.

4. Verfahren zum Identifizieren einer Zelleinheit und/oder Bestimmen einer Kulturbedingung, umfassend die Schritte:
(a) Bereitstellen eines ersten Satzes von Gruppen von Zelleinheiten, wovon jede eine oder mehrere Zellen umfasst, und Aussetzen der Gruppen gewünschten Zellkulturbedingungen;
(b) Mikroverkapseln der in Schritt (a) erhaltenen Zelleinheiten zusammen mit einem ersten Identifizierungsmarker;
(c) Vereinen von zwei oder mehreren in Schritt (b) erhaltenen Gruppen, um wenigstens einen Pool zu erhalten;
(d) Unterteilen des in Schritt (c) erhaltenen Pools, um einen weiteren Satz von Gruppen von Zelleinheiten zu bilden;
(e) Aussetzen der weiteren in Schritt (d) erhaltenen Gruppen von Zelleinheiten gegenüber unterschiedlichen Zellkulturbedingungen;
(f) Wiederholen der Schritt (b) bis (d) mit einem zweiten Identifizierungsmarker;
(g) Nachweisen der ersten und zweiten Identifizierungsmarker,
wobei die ersten und zweiten Marker verschieden ist.

5. Verfahren nach Anspruch 2 oder 3, wobei die Mikroverkapselung durch ein Jetting-Verfahren durchgeführt wird, welches gegebenenfalls ausgewählt ist aus der Gruppe bestehend aus Bio-Elektrospray-Jetting, aerodynamisch unterstütztem Bio-Jetting und druckunterstütztem Zell-Jetting.

6. Verfahren nach einem der Ansprüche 2 bis 4, wobei die Mikroverkapselung durch eine schichtweise Zugabe von Polyelektrolyten durchgeführt wird.

7. Verfahren nach einem der Ansprüche 2 bis 6, wobei die Zelleinheiten wenigstens einen Mikroträger umfassen.

8. Verfahren nach Anspruch 2 oder Anspruch 3, wobei die Kulturbedingungen ausgewählt sind aus der Gruppe bestehend aus Wachstumsmedien, Temperaturegimen, Substraten, atmosphärischen Bedingungen und physischer Zellhandhabung.

9. Verfahren nach einem der Ansprüche 1 bis 8, wobei im Wesentlichen jede Mikrokapsel eine einzelne Zelleinheit enthält.

10. Verfahren nach einem der Ansprüche 1 bis 8, wobei im Wesentlichen jede Mikrokapsel eine Vielzahl von Zelleinheiten enthält.

11. Verfahren nach einem der Ansprüche 1 bis 8, wobei jede Mikrokapsel eine Vielzahl von Zelleinheiten enthält, wobei jede Zelleinheit selbst individuell mikroverkapselt ist.

## Revendications

1. Procédé pour identifier une unité de cellule et/ou déterminer une condition de culture, comprenant les étapes suivantes :
(a) la fourniture d'une ou de plusieurs unités de cellule comprenant chacune une ou plusieurs cellules et l'exposition des unités de cellule à des conditions de culture souhaitées ;
(b) la microencapsulation de l'unité/des unités de cellule obtenue(s) dans l'étape (a) conjointement avec un ou plusieurs premiers marqueurs ;
(c) l'exposition de l'unité/des unités de cellule microencapsulée(s) obtenue(s) dans l'étape (b) à des conditions de culture souhaitées ;
(d) la répétition de la microencapsulation de l'étape (b) conjointement avec un second marqueur ; et
(e) la détection du premier marqueur et du second marqueur,
dans lequel les premier et second marqueurs sont différents.

2. Procédé pour identifier une unité de cellule et/ou déterminer une condition de culture, comprenant les étapes suivantes :
(a) la fourniture d'un premier groupe d'une ou de plusieurs unités de cellule comprenant chacune une ou plusieurs cellules et l'exposition des unités de cellule à des conditions de culture souhaitées ;
(b) la microencapsulation des unités de cellule obtenues dans l'étape (a) conjointement avec un premier marqueur d'identification ;
(c) le mélange des unités de cellule microencapsulées obtenues dans l'étape (b) avec une ou plusieurs unités de cellule qui ont été exposées à des conditions de culture différentes et microencapsulées avec un deuxième marqueur d'identification pour former un second groupe ;
(d) la répétition de l'étape (b) avec le second groupe, en utilisant un troisième marqueur d'identification ;
(e) la détection des premier, deuxième, et troisième marqueurs d'identification,
dans lequel au moins deux des premier, deuxième, et troisième marqueurs d'identification sont différents.

3. Procédé selon la revendication 2, dans lequel le premier groupe d'unités de cellule est séparé en deux ou plus de deux groupes avant ou après l'étape (c).

4. Procédé pour identifier une unité de cellule et/ou déterminer une condition de culture, comprenant les étapes suivantes :
(a) la fourniture d'un premier ensemble de groupes d'unités de cellule comprenant chacune une ou plusieurs cellules, et l'exposition desdits groupes à des conditions de culture souhaitées ;
(b) la microencapsulation des unités de cellule obtenues dans l'étape (a) conjointement avec un premier marqueur d'identification ;
(c) le regroupement de deux ou plus de deux groupes obtenus par l'étape (b) pour former au moins un regroupement ;
(d) la division du regroupement obtenu dans l'étape (c) pour créer un ensemble supplémentaire de groupes d'unités de cellule ;
(e) l'exposition des groupes supplémentaires d'unités de cellule obtenus dans l'étape (d) à des conditions de culture différentes ;
(f) la répétition des étapes (b) à (d) avec un second marqueur d'identification ;
(g) la détection des premier et second marqueurs d'identification,
dans lequel les premier et second marqueurs sont différents.

5. Procédé selon la revendication 2 ou la revendication 3, dans lequel la microencapsulation est effectuée par une procédure par jet, qui est sélectionnée facultativement dans le groupe constitué du bio-jet par électropulvérisation, du bio-jet assisté de manière aérodynamique et du jet de cellule assisté par la pression.

6. Procédé selon l'une quelconque des revendications 2 à 4, dans lequel la microencapsulation est effectuée par une addition couche par couche de polyélectrolytes.

7. Procédé selon l'une quelconque des revendications 2 à 6, dans lequel les unités de cellule comprennent au moins un microsupport.

8. Procédé selon la revendication 2 ou la revendication 3, dans lequel les conditions de culture sont sélectionnées dans le groupe constitué des milieux de culture, des régimes de température, des substrats, des conditions atmosphériques et de la manipulation physique des cellules.

9. Procédé selon l'une quelconque des revendications 1 à 8, dans lequel sensiblement chaque microcapsule contient une unique unité de cellule.

10. Procédé selon l'une quelconque des revendications 1 à 8, dans lequel sensiblement chaque microcapsule contient une pluralité d'unités de cellule.

11. Procédé selon l'une quelconque des revendications 1 à 8, dans lequel chaque microcapsule contient une pluralité d'unités de cellule, chaque unité de cellule étant elle-même individuellement microencapsulée.
